# EUROPEAN PATENT SPECIFICATION

(11) **EP 1 438 395 B1**
(45) Date of publication and mention of the grant of the patent: **30.11.2011**
(21) Application number: 02799523.2
(22) Date of filing: 27.09.2002
(51) Int. Cl.: C12N 5/07, A61K 35/39

(54) **GROWING XENOTRANSPLANT MATERIAL IN CULTURE**
ZÜCHTUNG VON FREMDTRANSPLANTATIONSMATERIAL IN KULTUR
CROISSANCE D'UNE MATIERE POUR XENOTRANSPLANT DANS UNE CULTURE

(30) Priority: 28.09.2001 NZ 51454601
(43) Date of publication of application: 21.07.2004
(73) Proprietor: Diabcell Pty Limited, Sydney NSW 2000 (AU)
(72) Inventor: GARKAVENKO, Olga, Auckland (NZ)
(74) Representative: Campbell, Patrick John Henry
(86) International application number: PCT/NZ2002/000197
(87) International publication number: WO 2003/027270

(56) References cited:
- WO-A-02/32437
- WO-A-97/16536
- WO-A-97/39107
- WO-A1-95/28167
- US-A- 6 149 907
- RAYAT G R ET AL: "Potential application of neonatal porcine islets as treatment for type 1 diabetes: a review" ANNALS OF THE NEW YORK ACADEMY OF SCIENCES, NEW YORK ACADEMY OF SCIENCES, NEW YORK, NY, US, vol. 875, 1999, pages 175-188, XP002962522 ISSN: 0077-8923
- RABINOVITCH A., RUSSELL T., MINTZ D.H.: 'Factors from fibroblasts promote pancreatic islet B cell survival in tissue culture' DIABETES vol. 28, no. 12, 1979, pages 1108 - 1113, XP008033545
- VERHOEVEN G. AND CAILLEAU J.: 'Influence of co-culture with sertoli cells on steroidogenesis in immature rat Leydig cells' MOL. CELL. ENDOCRINOL. vol. 71, 1990, pages 239 - 251, XP001199413
- MARIA-ENGLER S.S ET AL.: 'Microencapsulation and tissue engineering as an alternative treatment of diabetes' BRAZ. J. MED. BIOL. RES. vol. 34, no. 6, June 2001, pages 691 - 697, XP008033550

## Description

### FIELD

This invention generally relates to growing biological material by cell/tissue culture methods in order to make material suitable for transplanting into a mammal in order to overcome a disease, deficiency, or defect. More particularly the invention relates to preparations of cells and cell combinations for use in xenotransplantation, and specifically the invention relates to transplants of endocrine secretory cells such as islet cells for treating diabetes present within the recipient.

### BACKGROUND

A patient afflicted with the irreversible disease diabetes has defective islets of Langerhans and cannot produce sufficient insulin to respond appropriately to blood glucose increase. Since the discovery of insulin by Banting et al in 1922, exogenous (injected) insulin in repeated doses has comprised an effective method of control of blood glucose and has greatly extended the lives of many diabetics. Implants for use in controlling endogenous secretion defects such as diabetes are known, where ideally the implant would (a) produce sufficient insulin to avoid any need for use of injections or other means of administration of insulin, and also (b) assume a regulatory role, responding to swings in blood glucose concentration by producing a greater amount of insulin if the environment of the implant has an increased concentration of glucose. Kidney and pancreas, or pancreas alone allotransplantation is done from cadaver sources or sometimes live donors, but faces the usual problems of rejection involving immune responses. Islet transplantation after enzymic digestion of a cadaver pancreas is also known (e.g. Shapiro et al).

Xenotransplants of islet cells present a number of advantages, such as avoidance of certain diseases. For an xenotransplant, barriers of various types are used at least to confine the cells to a permanently identifiable, often subcutaneous site as well as to avoid an immune system challenge from the patient yet not detracting from the intended function. Specific pathogen free (SPF) pigs are suitable donors for xenografts of many types because of their anatomical and physiological similarities with humans and the porcine pancreas has been considered as a potential source of islets for xenotransplantation in diabetic patients. However, the high cost of SPF herd maintenance as well as the necessity to tightly control potential zoonoses has led to efforts to propagate islets *in vitro -* a kind of organ culture. Growing of animal cells *in vitro* for xenotransplantation if it were possible would provide many advantages including cost savings, and better control of the product, including better control of infectious pathogens such as potentially zoonotic viruses.

Previous research papers have shown only limited *in vitro* growth of adult and neonatal islets of different species. Neonatal tissue has a good capacity for growth in tissue culture, although a clear disadvantage of neonatal islet tissue is functional immaturity, which results in a period of 6-8 weeks before the grafted cells are able to regulate the hyperglycemia in diabetic recipients (see for example Korgsen O, Jansson L, Eizirik DL, Andersson A. "Functional and morphological differentiation of fetal porcine islet-like cells clusters after transplantation into nude mice". Diabetologia 1991; 34:379). It is also relatively difficult to hold differentiated cells (such as islet cells) in culture over long periods without reversion to a poorly differentiated version.

WO 97/39107 describes a method of increasing the maturation rate or proliferation rate of a cell utilizing microencapsulation techniques*.* Rayat et al ( Ann NY Acad Sci 1999; 875 : 175-188) describes the potential application of neonatal porcine islets as treatment for type I diabetes. There are many other forms of disease where the possibility of inserting extra material into the body in order to overcome the problem exists. For example, replacement of functions not or no longer available from other endocrine glands, the liver, or muscles or tendons, cartilage as blocks or other shapes for reconstruction, and lumps of bone.

One problem to be solved is to raise potentially insulin-secreting cells in a culture from, for example, neonatal porcine pancreatic tissue; including the step of maintaining the cells for an extended period. Another problem to be solved is to provide an effective xenotransplant capable of making an effective amount of insulin when exposed to hyperglycaemic conditions, despite the usual presence of a physical barrier intended to limit access of members of the immune system to the foreign cells.

### OBJECT

It is an object of this invention to create an improved cell preparation for use in xenotransplants, or at least to provide the public with a useful choice.

### DEFINITIONS

homologous serum: a serum for cell culture, taken from the same species of animal as that from which the cells were derived.
**artificial tissue:** a mixture, made *de novo* of more than one kind of living cell in tissue/cell culture, and after a period of time characteristically exhibiting (a) at least some physiological support of one kind of cell by another kind, and (b) at least some distinctive structural configuration including the cells of the mixture.
**connective tissue secretory cells;** a group including cells capable of secreting and supporting components of an extracellular matrix, such as chondrocytes, osteocytes, fibrocytes, including fibrocytes from tendons and fibrocytes from the dermis of the skin, and undifferentiated fibroblasts. This may include stem cells.
**endocrine secretory cells:** a group including, but not limited to: anterior pituitary cells, thyroid and parathyroid cells, pancreatic islet cells, adrenal cortical cells, ovarian and testicular cells including interstitial cells, choroid plexus cells, and the like.

Other first-type cells (as used herein) include any functional cell types including those of epithelial, connective tissue, or neural origin especially hepatocytes.
**support/trophic cells:** includes Sertoli cells, fibroblasts, some types of neuroglial cells, secretory cells including choroid plexus epithelial cells, and stem cells. Modified support/trophic cells include cells modified by alteration of their genetic composition so that they secrete a range of compounds similar to those secreted by, for example, Sertoli cells in a tissue culture environment and capable of promoting the growth and development of functional characteristics of secretory cells when grown in association.
**long-term:** a period of more than a week; typically extended to 4-6 weeks or more.

### STATEMENT OF INVENTION

In a first broad aspect this invention provides a method for carrying out long-term, over a period of 4 to 6 weeks or more, *in vitro* assembly, propagation and maturation of a functional artificial tissue comprised of more than one kind of cell taken from at least one species of mammal, wherein a selected first type of cell capable of being stimulated to secrete a product is co-cultured over the period together with at least one selected second type of cell capable of providing a support/trophic function for the first type, in a growth medium supplemented with an effective amount of homologous serum from the species of mammal that provided the first type of cell, so that organised functional, synergistic groups of mixed cell types are developed over the period, wherein the first type of cell is endocrine cells from pancreatic islets of Langerhans, and the second type of cell is selected from Sertoli cells or fibroblasts.

In a subsidiary aspect the invention provides a method as previously described in this section for making a functional artificial tissue wherein the artificial tissue includes the said selected first type of cell and the said second type of cell jointly capable of possessing a property, and is grown as a co-culture in a growth media supplemented with an effective amount of homologous serum, so that the artificial tissue may be grafted into a recipient suffering from a deficiency of the property in order to alleviate the deficiency.

Preferably the product is an endocrine hormone.

In a related aspect, the effective amount of homologous serum is in a range of from 5% to 15% by volume in the growth medium.

In a second related aspect, the growth media also includes nicotinamide within a range of from 5 mM to 15 mM.

Preferably at least the first type of cell is collected from a neonatal mammaL

The method of the invention can be used, for making a functional artificial tissue for use in alleviation of diabetes.

More particularly the artificial tissue is grown as a co-culture in a growth media supplemented with an effective amount of homologous serum, glucose, and nicotinamide, so that the resulting artificial tissue exhibits a functional secretory response to a local concentration of glucose, so that the artificial tissue may be grafted into a recipient suffering from diabetes in order to alleviate the disease.

In a third related aspect, the second type of cell is taken by biopsy from an individual in need of a functional graft and after *in vitro* propagation and maturation together with the first type of cell for a period the resulting co-culture is returned to the diabetic recipient in the form of a functional artificial tissue.

Preferably the second-type cells are fibroblasts, obtained from a specific individual intended as a subject for a subsequent xenografting procedure in which the individual receives subsequent generations of the fibroblasts as an allograft.

Typically, functional cell combinations have been grown as a co-culture for from 30 to 50 days in growth media of this type.

In a second broad aspect, the invention provides a functional artificial tissue comprised of more than one kind of cell taken from at least one species of mammal, wherein the functional artificial tissue is comprised of at least partially organised synergistic accumulations of cells comprised of a first type of cell, capable of being stimulated to secrete a product, and at least one second type of cell capable of providing a support/trophic function for the first type of cell as defined in claim 11.

Preferably the first type of cell is capable of expressing effective amounts of at least one secretion into the growth medium when in association with the second type of cell, so that the one or more secretions may be isolated from time to time for use.

In a related aspect, the functional artificial tissue is preserved for transport, storage and later use in a state of metabolic arrest.

In a further related aspect, the functional cells include cells capable of forming an extracellular matrix which may be shaped to suit a graft or transplant, including chondrocytes, osteocytes, fibrocytes from tendons, and the skin.

Preferably the effective amount of serum from the same species of mammal is at least 5% and more preferably is about 10%.

Preferably the effective amount of nicotinamide is at least 5 mM and more preferably is about 10 mM.

More preferably the invention provides an example tissue culture environment comprising Sertoli cells as a food layer for islet cells in the growth media known as RPMI 1640 media supplemented with 10% porcine serum, 10 mM nicotinamide, and 11.1 mM glucose (noting that these numbers are examples).

Preferably the Sertoli cells are from the same species of animal; more preferably they are from the same animal.

Preferably a ratio of seeded Sertoli cells to seeded islet cells is about 10,000 to 1 islet-equivalent (IEQ).

As alternatives to Sertoli cells as support/trophic cells, one may use fibroblasts.

In a second related aspect the invention provides functional cell combinations as previously described in this section, which have been grown together for at least 50 days in growth media of this type.

- alternatively the invention provides groups of islet cells which have been grown together for at least 30 days in growth media of this type.

In a third broad aspect the invention provides living material suitable for use in an xenotransplant; the material including groups of islet cells grown in an environment including (a) nicotinamide, (b) homologous (same species) serum, and (c) Sertoli cells all in a mutually effective amount.

Preferably the living material is encapsulated and a preferred encapsulation is carried out according to the Calafiore method.

Optionally the living material is provided in a metabolically slowed, or preserved state for transport or storage.

In a fourth broad aspect the invention provides a method of *in vitro* propagation and differentiation of functional islets in a medium as previously described in this section, using Sertoli cells as a matrix and a "nurse or feeder" layer.

Preferably the method includes the steps of
1. isolating Sertoli cells from a neonatal mammal
2. isolating pancreatic islet cells or progenitors thereof from a neonatal mammal,
3. forming a cultured layer of Sertoli cells on a surface, using a growth medium as herein described,
4. adding islet cells
5. and maintaining the cell combinations over a period, until the islet cells have differentiated into functional cells, and formed concentrated masses capable of secreting insulin at least partially in response to a glucose stimulus.

### PREFERRED EMBODIMENT

The description of the invention to be provided herein is given purely by way of example and is not to be taken in any way as limiting the scope or extent of the invention. The specific developments that have taken place during development of this invention are in relation to xenotransplantation for cases of diabetes. However it must be realised that the Examples to follow can be applied to other types of cells, such as hepatocytes, chondrocytes, and the like.

### ILLUSTRATIONS

- Fig 1.: Photomicrograph of a group of adjacent islets at various stages of development. A cell monolayer forms a background to a lower focal concentration of cells (normally after 5-7 days in culture), an upper focal formation of three-dimensional structures (normally at 2-3 weeks), and in the centre an islet-like structure (normally at 3-4 weeks in culture).
- Fig 2:: Islet cells (Liberase H isolation) cultured for 5 weeks with HSA.
- Fig 3:: Islet cells (Liberase H isolation) cultured for 5 weeks with 10% porcine serum (higher magnification than fig 2).
- Fig 4:: Islet cells (Collagenase P isolation) cultured for 5 weeks with HSA.
- Fig 5:: As fig 4 but with 10% porcine serum.
- Fig 6:: Sertoli cells cultured for 5 weeks with HSA.
- Fig 7:: As fig 6 but with 10% porcine serum
- Fig 8:: Islets with Sertoli cells 1:100 (1 islet equivalent to 100 Sertoli cells) cultured for 5 weeks with HSA.
- Fig 9:: As fig 8 but with 10% porcine serum.
- Fig 10:: Islets with Sertoli cells 1:10,000 cultured for 5 weeks with HSA
- Fig 11:: As fig 10 but with 10% porcine serum; resulting in large islets.
- Fig 12:: Islets/Sertoli cells (1:10,000) cultured for 5 weeks with porcine serum. Diameter of islets: 300 µm and 600 µm.
- Fig 13:: As Fig 12. Diameter of islet: 600 µm.
- Figs 14-17:: Comparison of sizes of islets from different cultures with 10% porcine serum: Fig 14: Islet (Collagenase P isolation) 250 µm, Fig 15: Islets/Sertoli 1:100 = 150 µm, Fig 16: Islets/Sertoli 1:10,000 = 500 µm. Fig 17: Islets/Sertoli cells cultured for 46 days. Size of the islet is 400 µm.
- Fig 18:: DTZ staining of Islets/Sertoli 1:10,000 with HSA (some positive staining)
- Figs 19 & 20:: DTZ staining of Islets/Sertoli 1:10,000 with porcine serum; 85% of cells positive to stain.
- Fig 21:: is a bar graph to show the insulin response of free islets after 46 days of culture and the effect of the presence of Sertoli cells during growth on the response.
- Fig 22:: is a bar graph to show the insulin response of encapsulated islets and the effect of the presence of porcine versus human serum during culture on the response, using a SGS test.

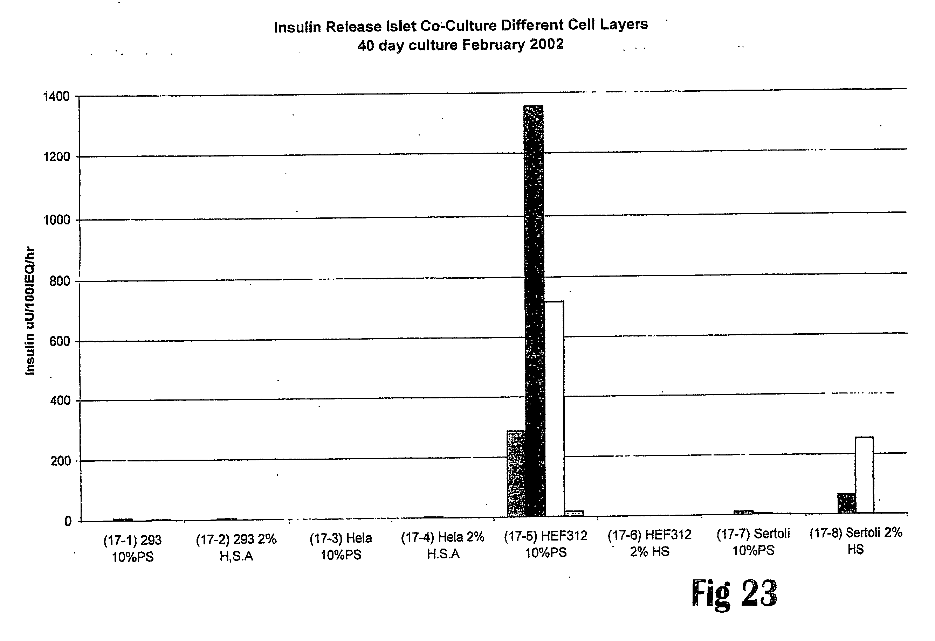

- Fig 23:: is a bar graph to show the insulin response of islet cells grown together with a variety of other cell types including the fibroblast line HEF312.

In general this invention relates to improvements in the preparation of cells for transplantation. One aspect of the improvements is the provision of better growth media for the cells during preparation for transplantation. Another aspect is the provision of a second cell type to form a supporting co-culture for the islet cells, and a preferred cell type exhibiting a combination of support and trophic effects, as it shows (among other functions) within the testis, is the Sertoli cell.

### EXAM PLE 1 a

This Example relates to tissue culture methods for raising and maturing cells intended for xenotransplantation.

**Porcine Islet Cell Isolation.** Pancreatic islets from 7 day old piglets were prepared following an adapted method from C. Ricordi (Pancreatic Islet cell Isolation. Austin R.G. Landes Co. 99:112, 1992 ). Our method includes enzymatic digestion (collagenase, liberase), culture for three days at 37 deg C in RPMI 1640 containing 2% human serum albumin, Ciproxin and 10 mmol/L nicotinamide in an atmosphere of 5% CO₂ and 95% air. Viability was tested with DTZ staining and insulin *release in vitro.*

**Porcine Sertoli Cell Isolation Cell Cultures in Combination.** Testicles from the same piglets were collected in HBSS solution including antibiotics. The isolation of Sertoli cells was done following the Rajotte procedure with some modifications (Rajotte, Diabetes, Vol 46, February 1997 317-322). The testicles were cleaned, separated from the capsule, and minced into 1 mm pieces with scissors. Digestion was done using DNA-ase and collagenase in a pre-heated waterbath at 37°C. Viability was tested using Sudan III and Trypan blue.

**Cell Cultures in Combination.** A set of flasks was maintained for 50 days, using a variety of media as per the following list. The media was changed weekly hence some components may change in concentration during the week. The base is RPMI 1640 media supplemented with either 2% HSA (human serum albumin or 10% porcine serum, 10mM nicotinamide, and 11.1 mM glucose, and the following combinations and variations were tested:
1. Control Islets with 2 % HSA
2. Islets with 10% porcine serum
3. Control Sertoli cells with 2 % of HSA.
4. Sertoli with 10% porcine serum
5. Control Islets /Sertoli 1:100 with 2% HSA.
6. Islets/Sertoli 1:100 with 10% porcine serum
7. Control Islets/Sertoli 1:10.000 with 2% HSA.
8. Islets/Sertoli 1:10.000 with 10% porcine serum

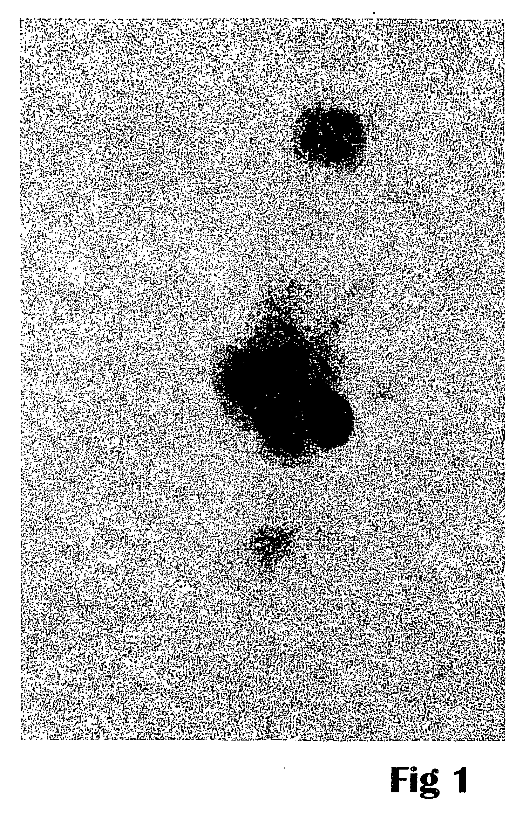

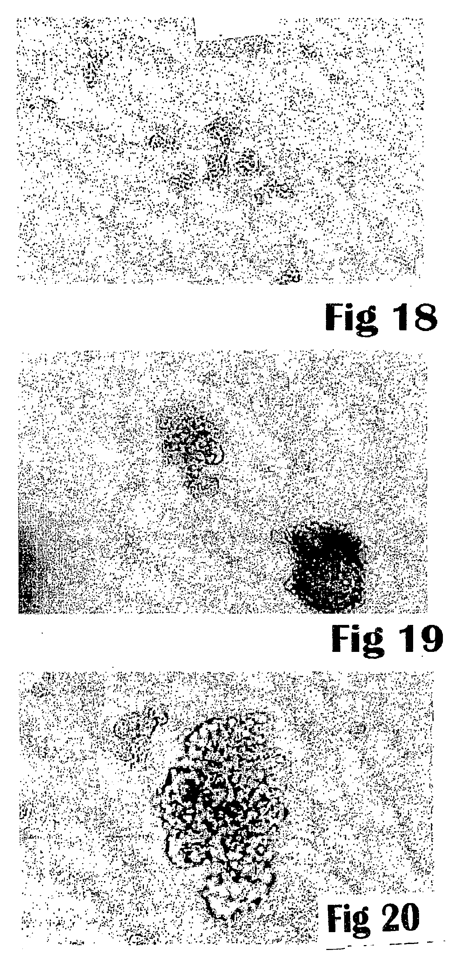

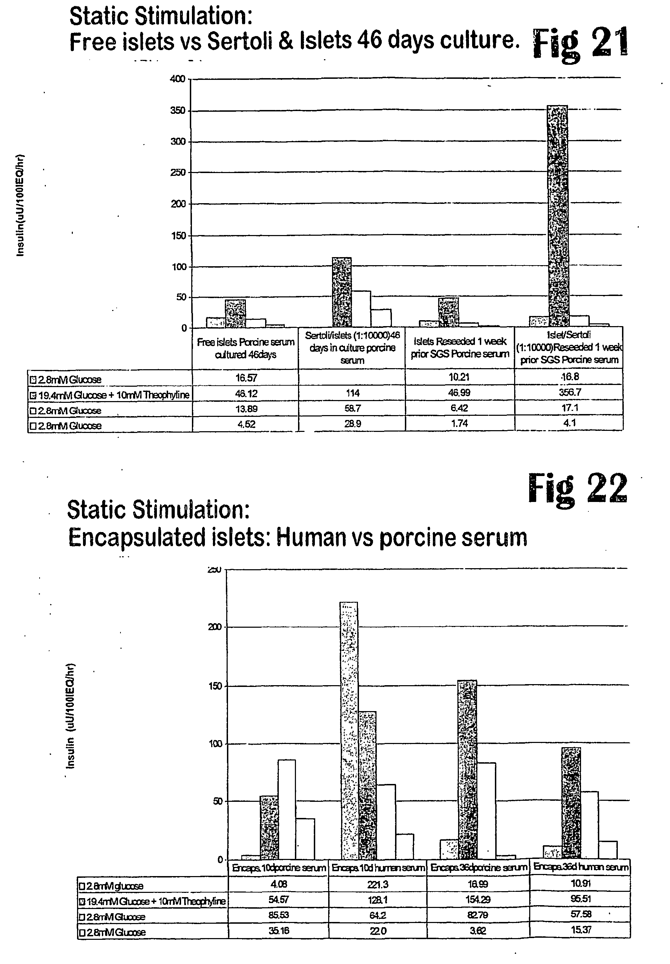

Samples were taken at day 46 for static stimulation with glucose (SGS) (Figs 21-22) and viability tests with DTZ (figs 18-20) and AO/PI staining. Results are expressed through photomicrographs Figs 1-20. Summary Fig 1 is a photomicrograph of a group of islets illustrating different stages of development close to each other, A cell monolayer forms a background to a focal concentration of cells as in the lower part of the picture (normally seen after 5-7 days in culture). In the upper part of this photomicrograph there is an early stage of formation of three-dimensional structures (normally seen at 2-3 weeks), and in the centre an islet-like structure (normally seen at 3-4 weeks in culture).

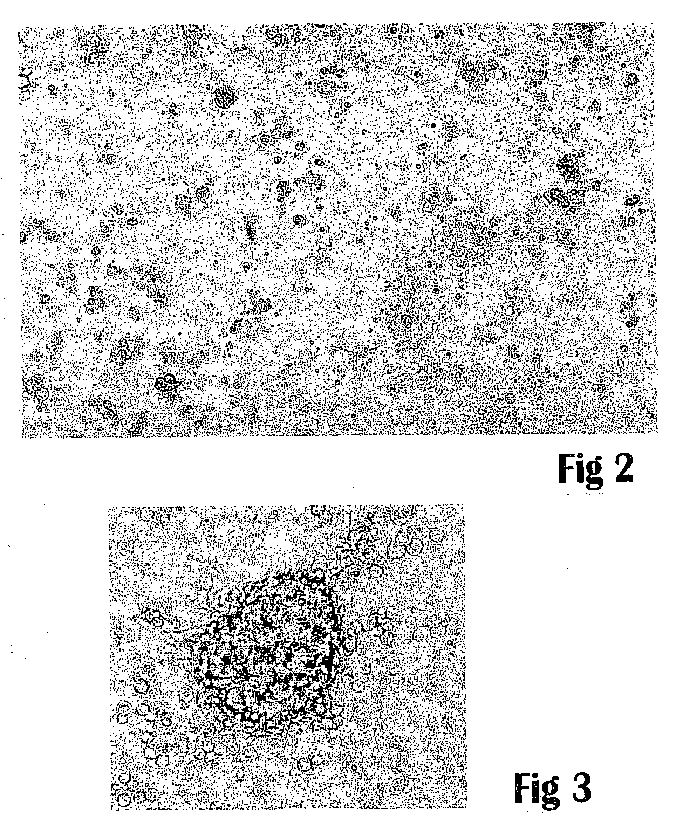

Compare fig 2 (islet cells (Liberase H isolation) cultured for 5 weeks with HSA) against fig 3 showing cells of the same origin cultured for 5 weeks with 10% porcine serum (at a slightly higher magnification than fig 2). An islet exists in the example where homologous (pig) serum is used. The human serum culture lacks evidence of growing cells.

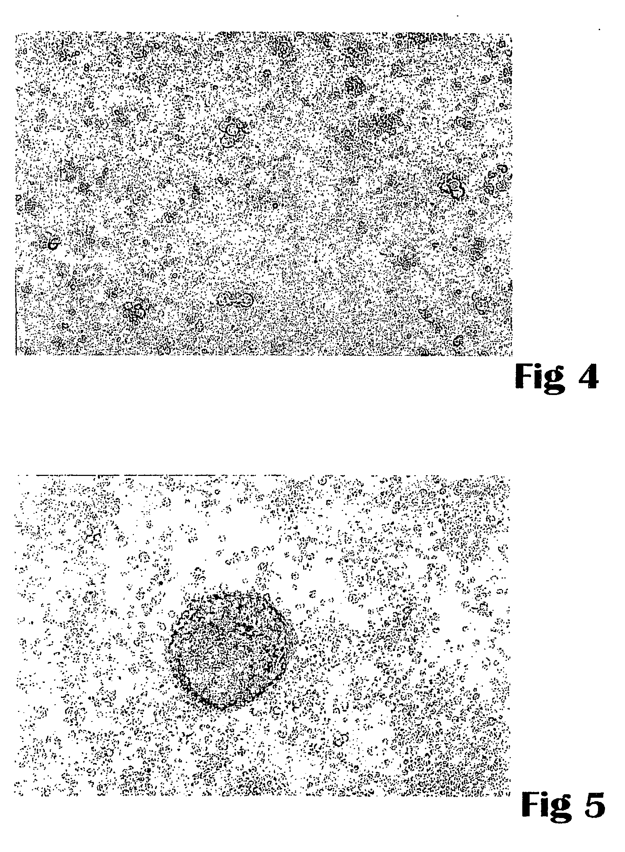

Compare fig 4 (Islet cells (Collagenase P isolation) cultured for 5 weeks with HSA) against fig 5 - the same but with 10% porcine serum. Again, an islet is present with homologous serum while the human serum culture appears to be moribund.

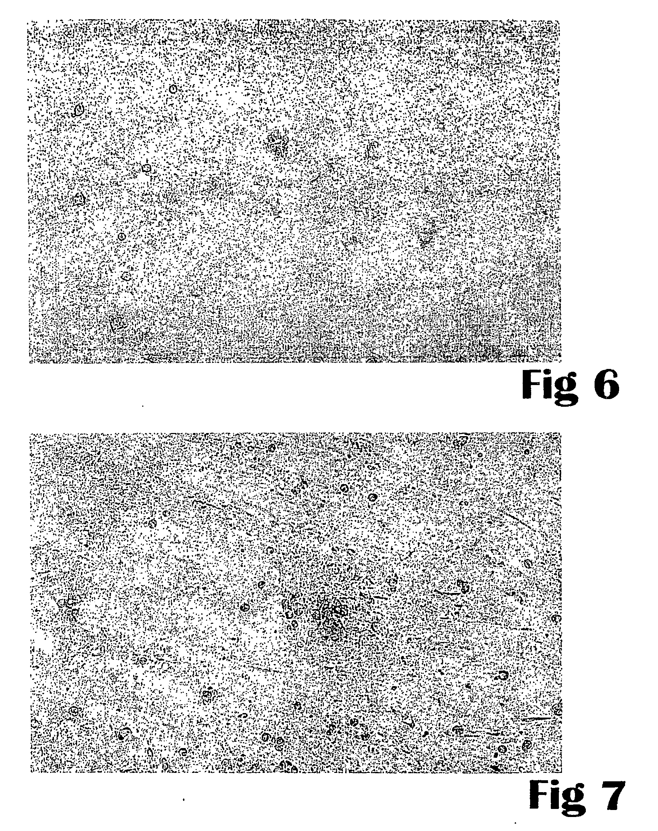

Compare fig 6 of Sertoli cells cultured for 5 weeks with HSA in the absence of islet cells against fig 7 (using 10% porcine serum); it appears that the homologous serum is beneficial to Sertoli cells - preceding Figures also showed that homologous serum is beneficial to islet cells. The next set examines co-cultures.

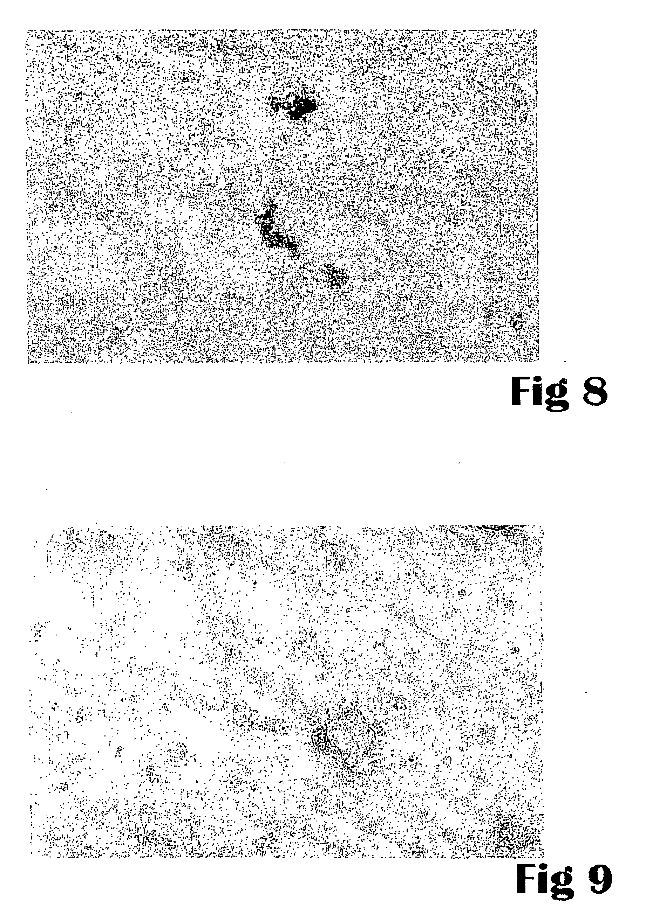

Compare fig 8 Islets with Sertoli cells in the ratio of 1:100 (1 islet equivalent for every 100 Sertoli cells) cultured for 5 weeks using HSA, against fig 9 (using 10% porcine serum). There is some cell activity in a co-culture with HSA but islet formation is seen only within the porcine serum flask.

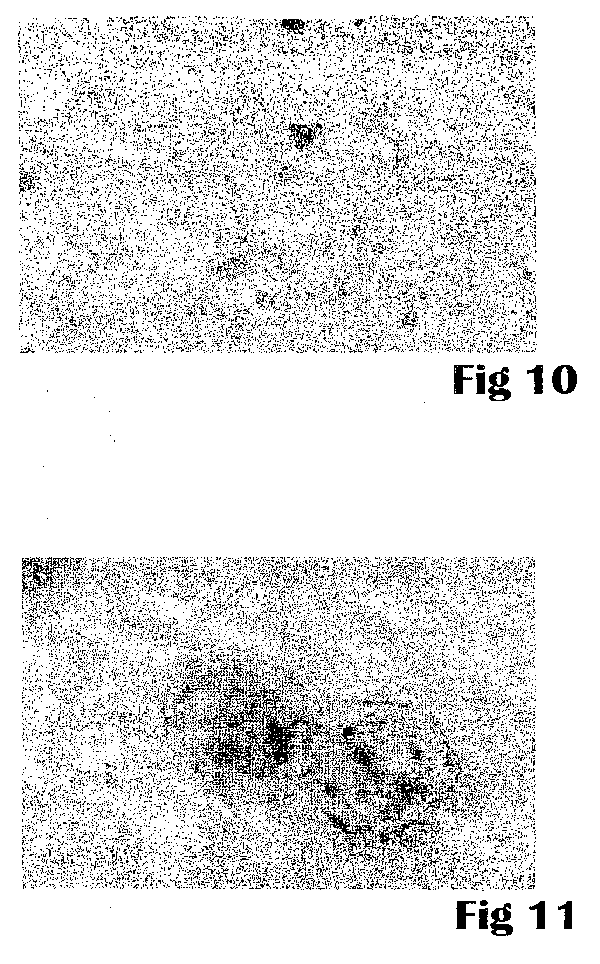

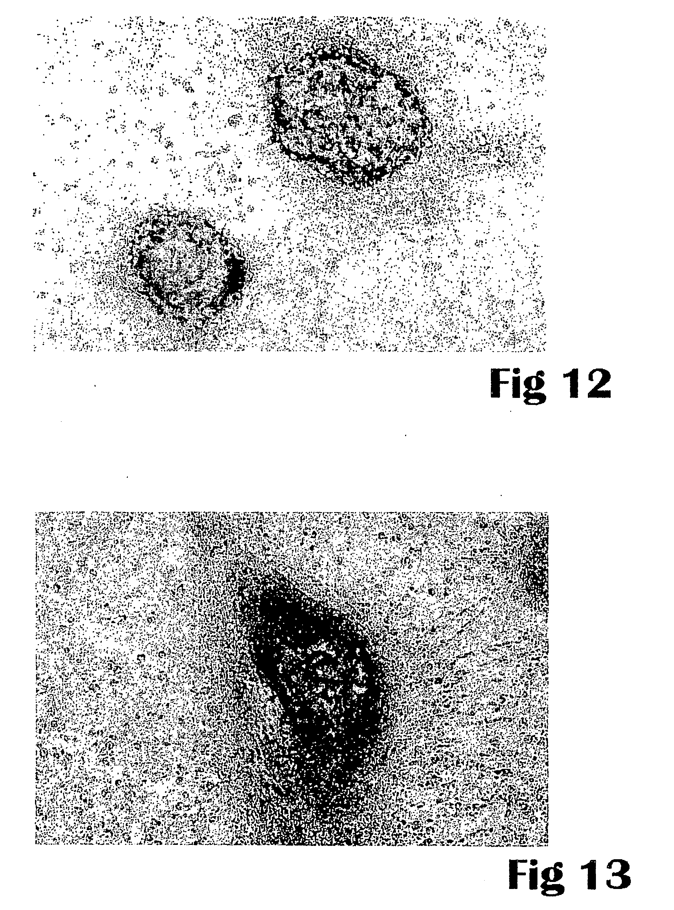

Using 1 islet equivalent (IEQ) for every 10,000 Sertoli cells appears to be more optimal. In fig 10 that ratio with human serum resulted in no islet formation; whereas in fig 11, 10% porcine serum results in large islets. Further examples using porcine serum at 10% are shown in Figs 12 and 13. Diameter of islets: 300 µm, 600 µm, and 600 µm.

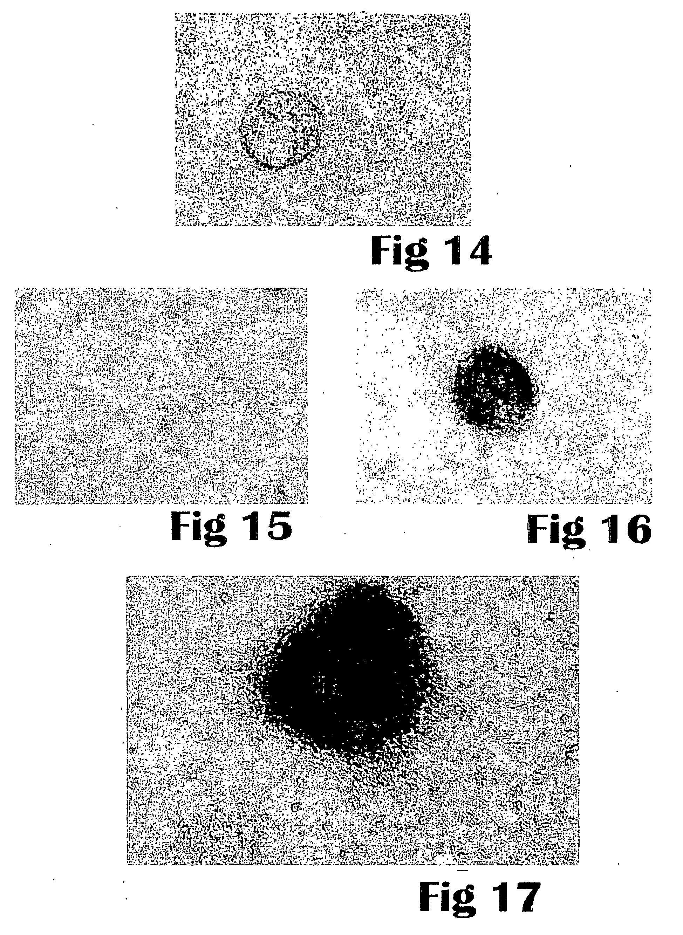

Figs 14 to 17 show various sizes of islets from differently treated cultures all raised with 10% porcine serum: Fig 14: Islet (Collagenase P isolation) 250 µm, Fig 15: Islets/Sertoli 1:100 = 150µm, Fig 16: Islets/Sertoli 1:10,000 = 500 µm. Fig 17: Islets/Sertoli cells cultured for 46 days and the size of this islet is 400 µm.

DTZ (dithizone) staining is shown in Figs 18, 19 and 20. Fig 18 grown under HSA (Islets/Sertoli 1:10,000) showed some positive staining. Figs 19 and 20 are two examples of (the same co-culture ratio with 10% porcine serum. Here at least 85% of cells stained positively for this test of insulin secretion.

One could conclude that there appears to be a synergistic co-operation between the use of homologous serum (in this case, 10% porcine serum) and co-culturing of islet cells with at least the Sertoli cells used in this Example, because only when both components are present is there formation of well-defined rounded islet structures as shown in many of the photomicrographs. This phenomenon could be described as "organogenesis" - if one can call an islet of Langerhans an organ. It appears that diameters of about 600 microns is the upper limit of size at least for the protocols tested to date, and this may be related to oxygenation of the cells at the centre of each ball.

### EXAMPLE 1b

**Insulin release tests (SGS test;** **Figs 21 & 22).** Fig 21 is a bar graph to show the insulin response of free islets after 46 days of culture and the effect of the presence of Sertoli cells during growth on the response. Fig 22 is a bar graph to show the insulin response of encapsulated islets and the effect of the presence of porcine versus human serum during culture on the response.

All the cells used in Fig 21 were cultured for 46 days in medium supplemented with 10% porcine serum. The maximal insulin release for free islets at day 46 was 46.1 uU/100 IEQ/hr compared to 114 uU/100 IEQ/hr when islets were co-incubated with Sertoli cells. Interestingly, the maximal insulin release was significantly higher when the mixture of Sertoli/islet cells was re-seeded one week before the SGS with a maximal insulin release of 356.7 uU/100 IEQ/hr compared to 46.99 uU/100 IEQ/hr from re-seeded islets not as a co-culture. Interestingly, islet cells in co-culture with Sertoli cells were able to produce some insulin even though there were lot of apoptotic cells (see photo 18). These results confirm that Sertoli cells preserve the beta-cell function.

The same islets were then encapsulated in alginate according to methods described by Calafiore et al (see Calafiore Basta & Boselli, Transplant Proc 1997; 29: 2126-7, (" Effect of alginate/polyaminoacidic coherent microcapsules (CM) transplantation in adult pigs") and Calafiore, Basta & Falorni, Diabetes Nutr Metab 1991; 4: 45-48 ("Vascular graft of microencapsulated human pancreatic islets in non immunosuppressed diabetic recipients; preliminary results") and cultured with human or porcine serum. The insulin release results are shown in Fig 22. "Static stimulation Encapsulated Islets: Human vs Porcine serum". The average maximal insulin release after 10 days was 54.57 uU/100 IEQ/hr for the encapsulated islets maintained with porcine serum compared to 128 uU/100 IEQ/hr when cultured with human serum. However, after 36 days in culture insulin release decreased to 54 uU/100 IEQ/hr for encapsulated islets with HAS and increased to 154 uU/100 IEQ/hr for encapsulated islets with porcine serum.

In order to demonstrate the effect of serum species on functional cell numbers, see the photomicrograph Fig 18: which is of a culture using Islets/Sertoli cells in a ratio of 1:10.000 with HAS. Only some islets still show some positive DTZ staining. In comparison, DTZ staining in Figs 19 and 20 with the same ratio of cell types but with porcine serum shows that more then 85% of the cells are DTZ positive cells.

It should be noted that we did not seek an immunological barrier comprised of Sertoli cells.

EXAM PLE 1 C DNA flow cytometry. A major concern for workers in this art is that the neonatally derived cells may undergo a potentially malignant transformation. Thus, for any long-term culture of cells-precursors the control of "normality" is very important. Flow cytometry has been used to check the ploidy of the cultured cells in this study. Table 1 represents the DNA content following different treatments.

**Table 1. DNA content in different experiment settings after 50 days in culture.**

| Treatment \ Resulting ploidy | **Diploid** | **Tetraploid** | **Aneuploid** |
|---|---|---|---|
| Islet cells with 2% HSA (*no events*) | 0 | 0 | 0 |
| Islet cells (Liberase H isolation) with 10% porcine serum | 93.6% | 4.91% | 0.44% |
| Islet cells ( Collagenase P isolation) with 10% porcine serum | 93% | 5% | 0.88% |
| Islet/Sertoli cells with 2% HSA | 0.03% | 0% | 0% |
| Islet/Sertoli cells with 10% porcine serum | 88.2% | 9.89% | 0.9% |
| PK15 (porcine embryo kidney cell line) as a same-species control | 66.6% | 24.9% | 3.72% |

### EXAMPLE 1 d

This Example relates to another tissue co-culture method for raising and maturing islets in combination with the recipient's own fibroblasts as obtained for example from a skin punch biopsy. Fibroblasts appear to be at least an equivalent to Sertoli cells. in terms of a synergistic supporting relationship, and the resulting cell groups are suitable for xenotransplantation. The recipient's fibroblasts (allocells) are used as a feeder layer and ideally would grow over the surface of a globule of islet cells during tissue culture and form a surface layer capable of at least partial immunoisolation so that the islet-fibroblast structures can be put into the recipient for insulin production. This isolation should prolong the effective life of the xenotransplant. This idea can be extended to the usage of any other recipient cells such as endothelial cells, chondrocytes etc.
**Experimental procedures.** Porcine islets isolated according to example 1a were put in co-culture with human primary (non-transformed) fibroblasts as well as with other cell types to compare the extent of proliferation and both amount and responsiveness of insulin release. The following cell types were used as a feeder layer:
1. Human embryonic kidney (HEK293) [ATCC CRL-1573]
2. HeLa line
3. Human fibroblasts (HEF312) [Auckland Hospital]
4. Porcine islet cells
5. Porcine Sertoli cells

Flasks with RPMI 1640 media supplemented with either 2% HSA or 10% porcine serum, 10mM Nicotinamide, and 11.1 mM glucose were maintained for 40 days:
1. Control Islets/human fibroblasts with 2 % H.S.A. (human serum albumin)
1. Islets/human fibroblasts with 10% porcine serum
2. Control islets/Sertoli cells with 2 % of H.S.A.
3. Islets/Sertoli with 10% porcine serum
4. Control Islets /HeLa with 2%H.S.A.
5. Islets/HeLa with 10%porcine serum
6. Control Islets/HEK293 with 2%H.S.A.
7. Islets/HEK293 1:10.000 with 10% porcine serum

The media was changed twice per week. After 40 days in co-culture, assessment of results was carried out. Photomicrographs were taken. Samples were also taken at day 40 for static stimulation with glucose (SGS) and DTZ staining. As shown in Fig the best result from the standardised SGS procedure was obtained from the islet/fibroblast co-culture, with about 1350 micro-units per 100 IEQ/hour, and the output of insulin dropped subsequently. Although this Example has not included the initial step of obtaining a large number of fibroblasts from a skin biopsy (we used a fibroblast line known as HEF312), the validity of the use of fibroblasts has been demonstrated.

### VARIATIONS

This Example may also relate to tissue culture methods for raising and bringing to functional maturity various cells and functional cell groups, other than islet cells, which are intended for xenotransplantation.

Note that the concentrations of porcine serum have been simply set at 10% without any attempt so far to ascertain an optimum amount. Nevertheless this Example covers such variations, just as it covers other concentrations of nicotinamide apart from the specified 10 mM. Further, the amount of glucose in the culture media used has not yet been the subject of experiment.

Apart from Sertoli cells, known to have an immunologically privileged position, it may be possible to use as trophic and supportive cells other types of cells including genetically modified cells, and unmodified neural support (neuroglial) cells, fibroblasts, adult stem cells, and chondrocytes.

Indeed, Example 1d supports the use of fibroblasts. There may be further benefit in using mixtures of Sertoli (or other types of) cells and fibroblasts together with porcine islet cells.

It may become ethically acceptable to maintain, for an individual, a bank of "spare cells" preserved in case of later need. This might include individuals at the onset of clinical diabetes, or individuals having a known susceptibility to a disease. It may also include homograft techniques wherein human cells (that is, Sertoli and islet cells or precursors thereof) are raised together, if needed, in the presence of human serum and the resulting islet cell groups may be encapsulated and returned to the individual. Incidentally the Sertoli cells may be from a different individual because it is unlikely that Sertoli cells will be included within the masses of islet cells that are encapsulated and used in transplants.

### COMMERCIAL BENEFITS or ADVANTAGES

Growing of animal cells *in vitro* for xenotransplantation (as compared to harvesting islets from mature animal pancreases) facilitates the procedure of encapsulated islet preparation, and would allow better control of infectious pathogens such as potentially zoonotic viruses, even retroviruses.

The invention also provides for the manufacture of cell secretions based on in vitro preparations, as an alternative to recombinant or synthetic manufacture. The advantage being that any glycosylation or the like of the peptide or proteins produced is provided by the mammalian cells themselves, and that any contaminating micro-organisms may be excluded from the products.

This invention provides a supply of islet cell groups suitable for encapsulation by known means and then for use in xenotransplants; the advantage of this invention being that the cell groups are larger, more active and available in greater quantities than was formerly possible.

Finally, it will be understood that the scope of this invention as described and/or illustrated within this provisional specification is not limited to the preferred embodiments described herein for illustrative purposes. Those of skill will appreciate that various modifications, additions, and substitutions are possible without departing from the scope of the invention as set forth in the following claims.

## Claims

1. A method for carrying out long-term, over a period of 4 to 6 weeks or more, *in vitro* assembly, propagation and maturation of a functional artificial tissue comprised of more than one kind of cell taken from at least one species of mammal, **characterised in that** a selected first type of cell capable of being stimulated to secrete a product is co-cultured over the period together with at least one selected second type of cell capable of providing a support/trophic function for the first type, in a growth medium supplemented with an effective amount of homologous serum from the species of mammal that provided the first type of cell, so that organised functional, synergistic groups of mixed cell types are developed over the period, wherein the first type of cell is endocrine cells from pancreatic islets of Langerhans, and the second type of cell is selected from Sertoli cells or fibroblasts.

2. A method as claimed in claim 1 for making a functional artificial tissue **characterised in that** the artificial tissue includes the said first type of cell and the said second type of cell jointly capable of possessing a functional property on being co-cultured in a growth media supplemented with an effective amount of homologous serum, so that the artificial tissue may be grafted into a recipient suffering from a deficiency of the property, to alleviate the deficiency.

3. A method as claimed in claim 1, **characterised in that** the product is an endocrine hormone

4. A method as claimed in claim 1, **characterised in that** the effective amount of homologous serum is in a range of from 5% to 15% by volume in the growth medium.

5. A method as claimed in claim 1, **characterised in that** the growth medium also includes nicotinamide within a range of from 5 mM to 15 mM.

6. A method as claimed in claim 1, **characterised in that** at least the first type of cell is collected from a neonatal mammal.

7. A method as claimed in claim 3 for making a functional artificial tissue **characterised in that** a ratio of the first type of cell to the second type of cell is optimised.

8. A method as claimed in claim 7 for making an artificial tissue **characterised in that** the artificial tissue is grown as a co-culture in a growth media supplemented with an effective amount of homologous serum, glucose and nicotinamide, so that the resulting artificial tissue exhibits a functional secretory response to a local concentration of glucose, so that the artificial tissue may be grafted into a recipient suffering from diabetes in order to alleviate the disease.

9. A method as claimed in claim 8 further **characterised in that** the second type of cell is taken from an individual in need of a functional graft and after *in vitro* propagation and maturation together with the first type of cell for a period is returned to the diabetic recipient in the form of a functional artificial tissue.

10. A method as claimed in claim 9 further **characterised in that** the functional cell combinations have been grown as a co-culture for from 30 to 50 days in growth media of this type.

11. A functional artificial tissue comprised of more than one kind of cell taken from at least one species of mammal, **characterised in that** the functional artificial tissue is comprised of at least partially organised synergistic accumulations of cells comprised of a first type of cell, capable of being stimulated to secrete a product, and at least one second type of cell capable of providing a support/trophic function for the first type of cell, the functional artificial tissue obtainable by a co-culture *in vitro* in a growth medium supplemented with an effective amount of homologous serum from the species of mammal that provided the first type of cell over a period of 4 to 6 weeks or more, wherein the first type of cell is endocrine cells from pancreatic islets of Langerhans, and the second type of cell is selected from Sertoli cells or fibroblasts.

12. A functional artificial tissue as claimed in claim 11, **characterised in that** the first type of cell is capable of expressing effective amounts of at least one secretion into the growth medium when in association with the second type of cell, so that the one or more secretions may be isolated from time to time for use.

13. A functional artificial tissue as claimed in claim 11, **characterised in that** the functional artificial tissue is preserved in a state of metabolic arrest suitable for transport, storage and later use.

## Patentansprüche

1. Verfahren zur Durchführung eines langfristigen, über einen Zeitraum von 4 bis 6 Wochen oder mehr, in vitro-Zusammenbaus, -Vermehrung und - Reifung eines funktionalen künstlichen Gewebes, bestehend aus mehr als einem Zelltyp, der aus wenigstens einer Spezies von Säugern entnommen ist, **gekennzeichnet dadurch, dass** ein ausgewählter erster Zelltyp, der zum Sekretieren eines Produkts stimulierbar ist, über einen Zeitraum zusammen mit wenigstens einem ausgewählten zweiten Zelltyp, der zur Bereitstellung einer fördernden/trophischen Funktion für den ersten Typ fähig ist, in einem Wachstumsmedium, ergänzt mit einer wirksamen Menge eines homologen Serums von der Spezies von Säugetier, die den ersten Zelltyp geliefert hat, ko-kultiviert wird, sodass organisierte funktionale, synergistische Gruppen von gemischten Zelltypen über den Zeitraum entwickelt werden, wobei der erste Zelltyp endokrine Zellen von pankreatischen Langerhansschen Inselns ist und der zweite Zelltyp ausgewählt ist aus Sertoli-Zellen oder Fibroblasten.

2. Verfahren nach Anspruch 1 zur Schaffung eines funktionalen künstlichen Gewebes, **gekennzeichnet dadurch, dass** das künstliche Gewebe den ersten Zelltyp und den zweiten Zelltyp gemeinsam umfasst, die zum Aufweisen einer funktionalen Eigenschaft bei Ko-Kultivierung in einem Wachstumsmedium, ergänzt mit einer wirksamen Menge an homologem Serum, fähig sind, sodass das künstliche Gewebe in einen Empfänger verpflanzt werden kann, der an einem Mangel der Eigenschaft leidet, um den Mangel zu verringern.

3. Verfahren nach Anspruch 1, **gekennzeichnet dadurch, dass** das Produkt ein endokrines Hormon ist.

4. Verfahren nach Anspruch 1, **gekennzeichnet dadurch, dass** die wirksame Menge des homologen Serums in einem Bereich von 5 bis 15 Volumenprozent in dem Wachstumsmedium liegt.

5. Verfahren nach Anspruch 1, **gekennzeichnet dadurch, dass** das Wachstumsmedium außerdem Nikotinamid innerhalb eines Bereichs von 5 mM bis 15 mM enthält.

6. Verfahren nach Anspruch 1, **gekennzeichnet dadurch, dass** zumindest der erste Zelltyp von einem neonatalen Säugetier entnommen ist.

7. Verfahren nach Anspruch 3 zur Schaffung eines funktionalen künstlichen Gewebes, **gekennzeichnet dadurch, dass** ein Verhältnis des ersten Zelltyps zu dem zweiten Zelltyp optimiert ist.

8. Verfahren nach Anspruch 7 zur Schaffung eines künstlichen Gewebes, **gekennzeichnet dadurch, dass** das künstliche Gewebe als eine Ko-Kultur in einem Wachstumsmedium, ergänzt mit einer wirksamen Menge an homologem Serum, Glukose und Nikotinamid, gezüchtet wird, sodass das resultierende künstliche Gewebe eine funktionale sekretorische Reaktion auf eine lokale Konzentration von Glukose zeigt, sodass das künstliche Gewebe in einen Empfänger verpflanzt werden kann, der an Diabetes leidet, um die Erkrankung abzumildern.

9. Verfahren nach Anspruch 8, außerdem **gekennzeichnet dadurch, dass** der zweite Zelltyp von einem Individuum entnommen ist, das eines funktionalen Transplantats bedarf, und nach der in vitro-Vermehrung und - Reifung gemeinsam mit dem ersten Zelltyp für einen Zeitraum dem diabetischen Empfänger in der Form eines funktionalen künstlichen Gewebes zurückgegeben wird.

10. Verfahren nach Anspruch 9, außerdem **gekennzeichnet dadurch, dass** die Kombinationen der funktionalen Zellen als eine Ko-Kultur für 30 bis 50 Tage in Wachstumsmedium diesen Typs gezüchtet worden sind.

11. Funktionales künstliches Gewebe, bestehend aus mehr als einer Art von Zellen, die aus wenigstens einer Spezies von Säugern entnommen sind, **gekennzeichnet dadurch, dass** das funktionale künstliche Gewebe sich aus zumindest teilweise organisierten synergistischen Anhäufungen von Zellen zusammensetzt, bestehend aus einem ersten Zelltyp, der zum Sekretieren eines Produkts stimulierbar ist, und wenigstens einem zweiten Zelltyp, der zur Bereitstellung einer fördernden/trophischen Funktion für den ersten Zelltyp fähig ist, wobei das funktionale künstliche Gewebe durch eine Ko-Kultur *in vitro* in einem Wachstumsmedium, ergänzt mit einer wirksamen Menge eines homologen Serums von der Spezies von Säugetier, die den ersten Zelltyp geliefert hat, über einen Zeitraum von 4 bis 6 Wochen oder mehr, erhältlich ist, wobei der erste Zelltyp endokrine Zellen von pankreatischen Langerhansschen Inselns ist und der zweite Zelltyp ausgewählt ist aus Sertoli-Zellen oder Fibroblasten.

12. Funktionales künstliches Gewebe nach Anspruch 11, **gekennzeichnet dadurch, dass** der erste Zelltyp zum Exprimieren wirksamer Mengen an wenigstens einer Sekretion in das Wachstumsmedium, wenn in Assoziation mit dem zweiten Zelltyp, fähig ist, sodass die ein oder mehreren Sekretionen von Zeit zu Zeit zur Verwendung isoliert werden können.

13. Funktionales künstliches Gewebe nach Anspruch 11, **gekennzeichnet dadurch, dass** das funktionale künstliche Gewebe in einem Zustand des metabolischen Stillstands, der für Transport, Lagerung und spätere Verwendung geeignet ist, konserviert ist.

## Revendications

1. Procédé pour opérer *in vitro,* sur une longue période, soit 4 à 6 semaines ou plus, l'assemblage, la croissance et la maturation d'un tissu artificiel fonctionnel constitué de cellules de plus d'un type, prélevées chez au moins une espèce de mammifère, **caractérisé en ce que** l'on met des cellules d'un premier type sélectionné, qui peuvent être stimulées pour sécréter un produit, en co-culture durant toute ladite période avec des cellules d'au moins un deuxième type sélectionné, capables d'assurer pour les cellules du premier type un rôle de support et/ou une fonction trophique, dans un milieu de croissance supplémenté avec une quantité efficace de sérum homologue issu de l'espèce de mammifère qui a fourni les cellules du premier type, de sorte que des groupes organisés, fonctionnels et synergiques de cellules mêlées de ces types se développent durant toute la période, et dans lequel procédé les cellules du premier type sont des cellules endocrines des îlots de Langerhans du pancréas et les cellules du deuxième type sont choisies parmi des cellules de Sertoli et des fibroblastes.

2. Procédé de fabrication d'un tissu artificiel fonctionnel, conforme à la revendication 1, **caractérisé en ce que** le tissu artificiel comprend des cellules dudit premier type et des cellules dudit deuxième type qui, après co-culture dans un milieu de croissance supplémenté avec une quantité efficace de sérum homologue, peuvent conjointement posséder une propriété fonctionnelle, si bien que le tissu artificiel peut être greffé chez un receveur souffrant d'un déficit en cette propriété, dans le but de remédier à ce déficit.

3. Procédé conforme à la revendication 1, **caractérisé en ce que** le produit est une hormone endocrine.

4. Procédé conforme à la revendication 1, **caractérisé en ce que** la quantité efficace de sérum homologue représente de 5 % à 15 % du volume du milieu de croissance.

5. Procédé conforme à la revendication 1, **caractérisé en ce que** le milieu de croissance contient aussi du nicotinamide, en une concentration valant de 5 mM à 15 mM.

6. Procédé conforme à la revendication 1, **caractérisé en ce qu'**au moins les cellules du premier type sont prélevées chez un mammifère nouveau-né.

7. Procédé de fabrication d'un tissu artificiel fonctionnel, conforme à la revendication 3, **caractérisé en ce que** le rapport des cellules du premier type aux cellules du deuxième type est optimisé.

8. Procédé de fabrication d'un tissu artificiel, conforme à la revendication 7, **caractérisé en ce qu'**on fait croître le tissu artificiel sous la forme de co-culture dans un milieu de croissance supplémenté avec une quantité efficace de sérum homologue, du glucose et du nicotinamide, de sorte que le tissu artificiel résultant offre une réponse sécrétoire fonctionnelle à une concentration locale de glucose, si bien que ce tissu artificiel peut être greffé chez un receveur souffrant d'un diabète, dans le but de remédier à cette maladie.

9. Procédé conforme à la revendication 8, **caractérisé en outre en ce que** les cellules du deuxième type sont prélevées chez un individu qui a besoin d'une greffe fonctionnelle et **en ce que**, après croissance et maturation *in vitro* subies conjointement avec des cellules du premier type durant une certaine période, elles sont réimplantées chez le receveur diabétique, sous la forme d'un tissu artificiel fonctionnel.

10. Procédé conforme à la revendication 9, **caractérisé en outre en ce que** l'on fait croître les combinaisons de cellules fonctionnelles sous forme de co-culture durant 30 à 50 jours, dans des milieux de croissance de ce type.

11. Tissu artificiel fonctionnel, constitué de cellules de plus d'un type, prélevées chez au moins une espèce de mammifère, **caractérisé en ce que** ce tissu artificiel fonctionnel est constitué d'accumulations de cellules, synergiques et au moins en partie organisées, constituées de cellules d'un premier type, qui peuvent être stimulées pour sécréter un produit, et de cellules d'au moins un deuxième type, capables d'assurer pour les cellules du premier type un rôle de support et/ou une fonction trophique, lequel tissu artificiel fonctionnel peut être obtenu par co-culture *in vitro* dans un milieu de croissance supplémenté avec une quantité efficace de sérum homologue issu de l'espèce de mammifère qui a fourni les cellules du premier type, durant une période de 4 à 6 semaines ou plus, et dans lequel tissu les cellules du premier type sont des cellules endocrines des îlots de Langerhans du pancréas et les cellules du deuxième type sont choisies parmi des cellules de Sertoli et des fibroblastes.

12. Tissu artificiel fonctionnel, conforme à la revendication 11, **caractérisé en ce que** les cellules du premier type sont capables d'exprimer, en quantités efficaces, au moins un produit de sécrétion dans le milieu de croissance quand elles s'y trouvent associées aux cellules du deuxième type, de sorte que l'on peut de temps en temps isoler le ou les produit(s) de sécrétion pour s'en servir.

13. Tissu artificiel fonctionnel, conforme à la revendication 11, **caractérisé en ce que** ce tissu artificiel fonctionnel est conservé dans un état où le métabolisme est arrêté, état approprié pour le transport, pour le stockage et pour une utilisation ultérieure.
